# EUROPEAN PATENT SPECIFICATION

(11) **EP 2 529 742 B1**
(45) Date of publication and mention of the grant of the patent: **20.11.2013**
(21) Application number: 11004534.1
(22) Date of filing: 03.06.2011
(51) Int. Cl.: A61K 31/7004, A61K 31/7034, A61K 47/48, A61P 3/10, A61K 31/424

(54) **Pharmaceutical composition comprising dapagliflozin and cyclodextrin**
Pharmazeutische Zusammensetzung mit Dapagliflozin und Cyclodextrin
Composition pharmaceutique comportant de la dapagliflozine et cyclodextrine

(43) Date of publication of application: 05.12.2012
(73) Proprietor: ratiopharm GmbH, 89079 Ulm (DE)
(72) Inventor: Puskas, István, 2111 Szada (HU); Szente, Lajos, 1118 Budapest (HU)
(74) Representative: Aechter, Bernd

(56) References cited:
- EP-A1- 2 233 134
- WO-A2-2008/131149
- WO-A2-2009/093264
- WO-A2-2011/060290

## Description

### Background of the invention

The present invention relates to pharmaceutical compositions comprising dapagliflozin and cyclodextrin, preferably as inclusion complexes, further preferably to molecularly dispersed dapagliflozin in a molecularly dispersed form of inclusion complexes, comprising cyclodextrin, preferably (2-hydroxy)propyl-β-cyclodextrin, and dapagliflozin. The invention further relates to a process for producing said pharmaceutical compositions and to dosage forms comprising said pharmaceutical compositions. "Dapagliflozin" is reported to be the INN name of the C-aryl glucoside compound (2S,3R,4R,5S,6R)-2-[4-chloro-3-(4-ethoxybenzyl)phenyl]-6-(hydroxymethyl)-tetrahydro-2*H*-pyran-3,4,5-triol and can be characterized by the following chemical formula:

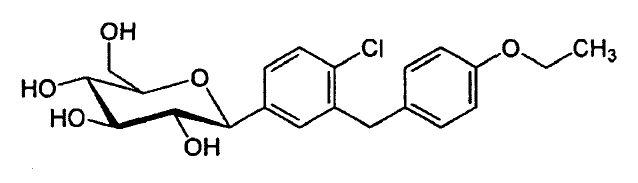

Dapagliflozin is reported to inhibit subtype 2 of the sodium-glucose transport proteins (SGLT2), which is responsible for at least 90% of the glucose reabsorption in the kidney. Blocking this transporter causes blood glucose to be eliminated through the urine. In particular, dapagliflozin is indicated for the treatment for type 1 and type 2 diabetes, in particular, type 2 diabetes.

Dapagliflozin and its synthesis are described in EP 1 506 211 B1. WO 2004/063209 A2 is directed to a process for preparing dapagliflozin and intermediates.

In the art, several dapagliflozin formulations are known. EP 1 506 211 B1 mentions combinations of dapagliflozin with an antidiabetic agent other than SGLT2 inhibitor, an agent for treating the complications of diabetes, an anti-obesity agent, an antihypertensive agent, an anti-platelet agent, an anti-atherosclerotic agent, and/or a lipid-lowering agent.

WO 2008/116179 A1 seems to disclose an immediate release formulation comprising dapagliflozin propylene glycol hydrate.

WO 2008/116195 A2 refers to the use of an SLGT2 inhibitor in the treatment of obesity, wherein the amount of said SGLT2 inhibitor is below the effective amount for treating diabetes.

WO 2011/060290 A2 discloses immediate release formulations comprising methformin and the sodium dependent glucose transporter (SGLT2) inhibitor dapagliflozin or its propylene glycol hydrate. Further, D1 describes methods for preparing said formulations and methods of treating diseases/disorders associated with SGLT2 activity employing these formulations.

WO 2009/093264 A2 discloses pharmaceutical combinations comprising an antidiabetic agent, wherein the antidiabetic agent may be a SGLT2 inhibitor, such as sergliflozin or dapagliflozin or pharmaceutically acceptable salt thereof.

WO 2008/131149 A2 discloses pharmaceutical combinations comprising one or more antidiabetic agent(s) selected from a plurality of active agents (inter alia dapaglflozin).

EP 2 233 134 A1 relates to a multi portion intra-oral dosage form comprising at least one pharmaceutically active agent or health promoting agent wherein at least one portion comprises a component for creating a noticeable organoleptic sensation. Further, document D4 is related to a pharmaceutically compound with cyclodextrin wherein the cyclodextrin is selected from α, β, γ-cyclodextrin.

The micronization of dapagliflozin, however, involves some disadvantages. Firstly, the micronization in the active ingredient results in an undesired low flowability. Furthermore, the micronized active ingredient is more difficult to be compressed and occasionally irregular distribution of the active ingredient can occur within the pharmaceutical formulation to be compressed. Caused by the large extension of the surface during micronization, the tendency of the active ingredient to oxidate increases. Moreover, dapagliflozin when provided in crystalline form is hygroscopic. Thus, it tends to attract water molecules from the surrounding environment through absorption, which leads to diffluence of the active substance, thereby impairing processing abilities and storage stability.

Hence, it was an object of the present invention to overcome the drawbacks of the above-mentioned formulations.

In particular, dapagliflozin should be provided in a form having superior solubility and processing abilities. Preferably, dapagliflozin should be provided in a form, which is highly soluble. In addition, dapagliflozin should be provided in a form, which allows oral application independently from meals. The increase in solubility and permeability should particularly be achieved without a micronization step and preferably without the use of excipients or co-solvents. Moreover, dapagliflozin should be provided in a non-hygroscopic form.

Furthermore, it was an object of the invention to provide dapagliflozin in a form having superior storage properties. Preferably, storage stability for 12 months at 40 °C and 75 % humidity should be achieved. After storage under said conditions, impurities should be less than 2 wt.-%, more preferably less than 1 wt.-%.

Particularly, the above-mentioned objects should be solved simultaneously, that means, dapagliflozin should be provided in a non-hygroscopic form having high solubility, high permeability and showing high storage stability.

### Summary of the invention

The objects of the present invention can be solved by pharmaceutical compositions comprising dapagliflozin and cyclodextrin, preferably as dapagliflozin cyclodextrin inclusion complex, more preferably as "genuine" dapagliflozin-cyclodextrin inclusion complexes and a method for forming such pharmaceutical compositions. Preferably, said preferred inclusion complex can be regarded as a supramolecular, non-covalent inclusion complex. The preferred "genuine" inclusion complex can lead to a novel solid form of dapagliflozin, preferably to a form of molecular dispersity, and, when rewetted, in a form having amorphous structure. The novel solid form can be described as glassy-amorphous solid form.

A subject of the present invention thus can be a pharmaceutical composition comprising dapagliflozin and cyclodextrin, preferably as an inclusion complex.

A preferred subject of the present invention can be a inclusion complex comprising (2-hydroxy)propyl-β-cyclodextrin (HPBCD), sulfobutylether-β-cyclodextrin (SBEBCD) and/or (2-hydroxy)-propyl-γ-cyclodextrin (HPGCD), in particular (2-hydroxy)propyl-β-cyclodextrin (HPBCD) and dapagliflozin.

The above illustrated subjects of the present invention are alternative solutions to the above outlined objects.

Moreover, the present invention can be directed to a process for producing a pharmaceutical composition comprising dapagliflozin and cyclodextrin - preferably in form of an inclusion complex - comprising the steps of
a) dissolving and/or dispersing cyclodextrin in a solvent;
b) adding dapagliflozin;
c) subjecting the mixture resulting from step (b) to a mechanical treatment; and
d) removing the solvent from the reaction mixture, preferably by freeze-drying or spray-drying.

In a further embodiment, the present invention can be related to the use of cyclodextrin, preferably (2-hydroxy)propyl-β-cyclodextrin, for producing a dapagliflozin-containing dosage form.

Finally, the present invention can be directed to the method of purification of dapagliflozin, comprising the steps of
a) dissolving and/or dispersing cyclodextrin in a solvent;
b) adding crude dapagliflozin;
c) separating the complexed dapagliflozin from non-complexed residues.

### Detailed description of the invention

The term " dapagliflozin " as used in the present application refers to dapagliflozin in free form as well as to its pharmaceutically acceptable solvates, hydrates, enantiomers, polymorphs or mixtures thereof. Dapagliflozin can be further provided as hydrates or solvates, for example propylene glycol hydrate (PGS). Preferably, dapagliflozin can be used in the non-solvated form.

In a preferred embodiment of the invention, the present invention can refer to a "genuine" dapagliflozin-cyclodextrin inclusion complex. The term "genuine" indicates that the entire and complete amount of dapagliflozin can be entrapped intercalated into the molecular cavities of the cyclodextrin, i.e. dapagliflozin is only present in intercalated form. No adsorbed, un-entrapped crystalline or amorphous dapagliflozin occurs. The formation of the preferred "genuine" inclusion complex generally can lead to a glassy-amorphous solid form of dapagliflozin.

Preferably, the inclusion complexes of the present invention can be non-covalent inclusion complexes. Furthermore, the inclusion complexes of the present invention can be supramolecular inclusion complexes. In particular, the inclusion complexes of the present invention can be non-covalent and supramolecular inclusion complexes. The term "supramolecular" is understood as describing self-organizing molecular interactions that result in the formation of new structures that stay together without establishing a covalent linkage.

Generally, in the pharmaceutical composition of the present invention, prefereably in the dapagliflozin-cyclodextrin inclusion complexes, the molar ratio of cyclodextrin to dapagliflozin is from from 0.5 : 1 to 2 : 1, more preferably from 0.8 : 1 to 1.2 : 1, most preferably 1.0 : 1.0. Thus, it is particularly preferred that the dapagliflozin functions as "monofunctional guest" within the cyclodextrin cavity.

The term "cyclodextrin" generally refers to non-reducing cyclic saccharides. Preferably, said cyclic saccharides comprise six, seven or eight glucose units linked by alpha-1,4 interglycosidic bonds. In the present invention cyclodextrins comprising seven glucopyranose units (cycloheptaamylose) are preferred.

The cyclodextrin can be a naturally occurring cyclodextrin or a chemically modified cyclodextrin. Preferably, the cyclodextrin is selected from α-cyclodextrin (ACD), β -cyclodextrin (BCD), 2-hydroxypropyl-β-cyclodextrin (HPBCD), randomly methylated β-cyclodextrin, sulfobutyl ether-β-cyclodextrin (SBEBCD), and 2-hydroxypropyl-γ-cyclodextrin (HPGCD).

Furthermore, it is particularly preferred that in all embodiments of the present invention cyclodextrins can be used in the form of cyclodextrin hydrate, particularly (2-hydroxy)propyl-β-cyclodextrin, sulfobutylether-β-cyclodextrin and/or (2-hydroxy)-propyl-γ-cyclodextrin, most preferably (2-hydroxy)propyl-β-cyclodextrin can be used in the form of the hydrate, such as (2-hydroxy)propyl-β-cyclodextrin hydrate. In a preferred embodiment the water content of the cyclodextrin used for making the inclusion complex can be less than 15 wt.-%, preferably 1 to 12 wt.-%, most preferably 1 to 8 wt.-%, based on the total weight of the cyclodextrin.

The cyclodextrins of the present invention can be (partially) substituted. Substitution can be achieved with acetyl groups, alkoxy groups such as carboxymethyl, heteroaromatic or aromatic groups such as benzyl, heteroalkyl or alkyl groups, preferably C₁-C₈ alkyl groups such as methyl, ethyl, propyl, butyl and pentyl, or with hydroxyalkyl groups such as hydroxyethyl and hydroxypropyl. Preferably, the cyclodextrins can be (partially) substituted with hydroxypropyl groups.

The average degree of substitution is usually 0.1 to 3, preferably 0.3 to 2, more preferably 0.4 to 1.5 and most preferably 0.5 to 1 per glucose unit. In case (2-hydroxy)propyl-β-cyclodextrin can be used, the degree of substitution is 0.1 to 2, preferably 0.3 to 1.5 and most preferably 0.5 to 1 of hydroxypropyl groups per glucose unit.

In an alternative preferred embodiment, the pharmaceutical composition of the invention, preferably the dapagliflozin-cyclodextrin inclusion complex, comprises dapagliflozin and sulfobutylether-β-cyclodextrin and/or (2-hydroxy)propyl-β-cyclodextrin.

Generally, the pharmaceutical composition of the present invention, preferably the "genuine" dapagliflozin-cyclodextrin inclusion complex can be achieved if (2-hydroxy)propyl-β-cyclodextrin is used. (2-hydroxy)propyl-β-cyclodextrin is a ringshaped molecule made up of seven glucose units linked by alpha-1,4 bonds, wherein the glucose units can be (partially) substituted with hydroxypropyl groups.

Furthermore, it is preferred that (2-hydroxy)propyl-β-cyclodextrin (HPBCD) is used in the form of a hydrate, wherein each molecule of (2-hydroxy)propyl-β-cyclodextrin comprises between 12 and 14 molecules of water. In addition, crystalline (2-hydroxy)propyl-β-cyclodextrin, having a monoclinic space group, is used.

Generally, cyclodextrins form an inner cavity. Within this application said cavity can be referred to as "nanocavity".

The "genuine" dapagliflozin-cyclodextrin inclusion complex of the present invention can be characterized by the complete inclusion of the dapagliflozin molecule into the nanocavity of the cyclodextrin(s). The completeness of the inclusion process can be monitored via solid state Raman microscopy/spectroscopy and/or by the SEM-ESD electron-microscopic surface mapping.

The preferred "genuine" dapagliflozin-cyclodextrin inclusion complexes of the present invention can be characterized by the complete inclusion of the dapagliflozin into the cavity of the cyclodextrin. The term *"complete inclusion*" means that the molecular entrapment of dapagliflozin is essentially quantitative. In other words, preferably no surface-bound, adsorbed fraction of the dapagliflozin will occur in the dapagliflozin/cyclodextrin, particularly in the preferred dapagliflozin/beta-cyclodextrin complexes according to the present invention.

Hence, in a preferred embodiment of the invention the pharmaceutical composition comprising dapagliflozin and cyclodextrinpreferably can form a solid phase being essentially free of crystalline dapagliflozin. The term "essentially" free means that the preferred inclusion complexes of the present invention do not contain significant amounts of crystalline dapagliflozin bound to the surface of complex particles. Preferably, the pharmaceutical composion of the present can comprise less than 5 wt.-%, more preferably less than 2 wt.-%, more preferably less than 0.5 wt-% dapagliflozin in crystalline, based on the total weight of the pharmaceutical composition.

In a preferred embodiment the residual water content of the preferred dapagliflozin-cyclodextrin inclusion complexes of the present invention is 0.01 to 8 wt.%, more preferably 0.1 to 7.5 wt.%, still more preferably 0.3 to 7.0 wt.%, most preferably 0.5 to 6.5 wt.%, based on the total weight of the complex. Preferably, the water content of the preferred dapagliflozin-cyclodextrin complexes of the present invention does not increase by more than 6 wt.% (in particular, not more than by 3 wt.%) during a storage period of 3 months, at a temperature of 25 °C and a humidity of 60 %.

The residual water content is determined according to the Karl Fischer method. For the titration a Mettler Toledo DL - 31 titrator is used and the calculation of the water content is performed by the apparatus itself. Usually, a sample of about 50 mg of dapagliflozin cyclodextrin complex is analyzed.

The average particle size of the dapagliflozin-cyclodextrin inclusion complexes, preferably of the (2-hydroxy)propyl-β-cyclodextrin dapagliflozin inclusion complexes, is usually between 2 and 20 micrometers, preferably between 5 to 15 micrometers and particularly between 6 and 12 micrometers.

The term "average particle size" refers to the volume average particle size (D₅₀), which can be determined by the light scattering method, using a Mastersizer 2000 apparatus, made by Malvern Instruments (wet measurement, 2000 rpm, ultrasonic waves for 60 sec., data interpretation via Fraunhofer method).

Furthermore, the pharmaceutical composition of the present invention - preferably in form of the inclusion complex - can be provided in a solid, particulate form having a bulk density of 450 to 900 mg/cm³, more preferably from 500 to 880 mg/cm³.

The dapagliflozin-cyclodextrin inclusion complexes of the present invention can be regarded as a novel glassy-amorphous solid phase of dapagliflozin. The glassy-amorphous solid phase of dapagliflozin, re-wetted in an aqueous system, preferably can show some liquid crystalline properties that remind of the lyotropic liquid crystalline material. In contrast, the bulk solid was found to have an amorphous structure and is preferably entirely amorphous. Thus, the bulk solid of the dapagliflozin inclusion complex is preferably more than 98 wt.%, more preferably more than 99 wt.% amorphous, most preferably more than 99.5 wt.% amorphous.

The present invention further relates to a process for producing a pharmaceutical composition comprising dapagliflozin and cyclodextrin. preferably as "genuine" non-covalent dapagliflozin-cyclodextrin inclusion complex. Hence, a further subject of the present invention can be a process for producing a pharmaceutical composition comprising dapagliflozin and cyclodextrin,- preferably in form of an inclusion complex - comprising the steps of
a) dissolving or dispersing cyclodextrin in a solvent;
b) adding dapagliflozin;
c) subjecting the mixture resulting from step (b) to a mechanical treatment; and
d) removing the solvent from the reaction mixture, preferably by freeze-drying or spray-drying.

Generally, the comments made above for dapagliflozin and cyclodextrin can also apply to the process of the present invention. Thus, for example, preferably β- -cyclodextrin can be used, particularly (2-hydroxy)propyl-β-cyclodextrin (particularly having the above illustrated water content) can be used in the process of the present invention.

In step a) of the process of the invention cyclodextrin can be dissolved or dispersed in a suitable solvent. The solvent may be water or an organic solvent, preferably an alcohol, for example ethanol. Furthermore, the solvent may also be a mixture of alcohol and water, wherein the mixing ratio alcohol : water is for example 1 : 10 to 2 : 1. In a particularly preferred embodiment the solvent can be deionized water.

The term "dissolving or dispersing" means that cyclodextrin is brought into contact with the solvent, preferably with the water, wherein the solvent wets the surface of the cyclodextrin or the cyclodextrin can be dispersed (i.e. suspended and optionally partially dissolved) in the solvent or, in a preferred embodiment, the cyclodextrin can be completely dissolved in the solvent.

The weight ratio of cyclodextrin : solvent can range from 1 : 10 to 10 : 1, preferably from 1 : 1 to 1 : 5.

Optionally, the cyclodextrins can be stirred during the dissolving or dispersing step, preferably at a stirring speed from 300 to 450 rpm (rotation per minute).

In an optional but preferred embodiment, dapagliflozin can be subjected to a grinding step (referred to as step (aa)). The grinding step (aa) can lead to a kind of "activation" of the dapagliflozin. Usually, grinding can be carried out for 1 to 30 minutes, preferably for 2 to 10 minutes. Grinding in step (aa) may be carried out in known milling devices, for example a ball mill.

Optionally, dapagliflozin can be (partially or completely) dissolved in a co-solvent (referered to as step (ab))

In step b) of the process dapagliflozin can be added, preferably in particulate form. More preferably, dapagliflozin can be added in crystalline solvated form to the solution of step a). The molar ratio of cyclodextrin to dapagliflozin can be preferably from 1 : 1 to 5 : 1, more preferably from 1 : 1 to 4 :1, particularly from 1 : 1 to 3 : 1.

Optionally, the mixture of step b) can be stirred, preferably at a stirring speed from 300 to 450 rpm. Stirring may be carried out for 1 to 10 minutes.

In step c) the mixture resulting from step b) can be subjected to a mechanical treatment.

Generally, any mechanical treatment can be suitable to enable the inclusion of the dapagliflozin into the nanocavity. Preferably, the mechanical treatment step can comprise ultrasonic treatment, optionally combined with stirring. Alternatively, but also preferred, mechanical treatment can be carried out by grinding, preferably by co-grinding wetted and/or dispersed cyclodextrin with dapagliflozin.

Generally, ultrasonic treatment can be carried out by immersing the mixture resulting from step b) into an ultrasonic device, for example, an ultrasonic bath. Examples of ultrasonic-treatment are hydrodynamic cavitation, sono-fragmentation and/or sono-cavitation or co-grinding. For example, ultrasonic treatment can be carried out with Tesla ultrasonic equipment.

Ultrasonic treatment can preferably be performed by using ultrasonic waves having a frequency of 5 to 100 kHz, more preferably of 10 to 80 kHz. Furthermore, ultrasonic treatment is preferably performed by using ultrasonic waves having an intensity of 50 to 5000 W, more preferably 500 to 1000 W. As an example, 1000 W and 20 kHz or 500 W and 58 kHz can be used.

This means that instead of the relatively long 6-8 hours stirring time for reaching complete inclusion, which is used traditionally in the art for complexation, the strirring time can significantly be reduced by the above-mentioned sono-fragmentation or sono-cavitation process. By this high-energy ultrasonic treatment, the inclusion complexation technology can become more efficient and economic.

In addition to the ultrasonic treatment (for example hydrodynamic cavitation, sono-fragmentation or sonocavitation), the reaction mixture may be agitated (for example using traditional propeller stirrer), preferably with a rotation speed of 300-450 rpm (rotation per minute).

As mentioned above, the mechanical treatment step can also be carried out by grinding preferably by co-grinding dispersed cyclodextrin with dapagliflozin. Generally, grinding can be carried out in known milling devices, for example a ball mill or a pin mill. It is preferred that if the mechanical treatment step c) is carried out by grinding, then the optional step (aa) (= activation of cyclodextrin) is carried out.

Usually, the mechanical treatment can be carried out for 1 to 30 minutes, preferably for 5 to 20 minutes. Furthermore, mechanical treatment can be carried out at a temperature from 5 to 50 °C, preferably at room temperature (about 20 °C).

Once solid phase transformation (occuring in step c)) is completed, the solvent of the reaction mixture can be removed in step d).

Generally, the methods known in the art for removing solvents are suitable. Preferably, the solvent can be removed by freeze-drying or spray-drying. The removal of the solvent by a spray-drying step is particularly preferred.

Generally, spray-drying can be carried out, using an inlet temperature of 150 to 200 °C, preferably about 180 °C, and an outlet temperature of about 80 to 100 °C. preferably of about 95 °C. For example, spray-drying can be carried out by using a Büchi^{®} Lab Niro spray-drier.

After removing the water, the pharmaceutical compositions of the present invention, preferably the dapagliflozin-cyclodextrin inclusion complexes, can be rewetted, dispersed or dissolved. The fastest dissolving form of the dapagliflozin inclusion complexes of the present invention usually can be achieved, if in step d) the solvent is removed by freeze-drying (liophilisation) the inclusion complex.

Steps a) to c) can be carried out subsequently or simultaneously. In a preferred embodiment steps a), b) and c) can be carried out subsequently.

Generally, the process of the present invention is suitable for preparing the inclusion complexes of the present invention, preferably achieving a yield of from 80 to 99 %, more preferably from 90 to 98 %.

In a preferred embodiment of the process of the invention the pharmaceutical composition comprising dapagliflozin and cyclodextrin can be formed in the absence of excipinets and/or co-solvents.

Further subjects of the present invention can be pharmaceutical compositions, preferably as inclusion complexes, obtainable by the above mentioned process. The pharmaceutical compositons of the present invention, preferably as inclusion complexes can be applied in the form of a dosage form.

Hence, a further subject of the present invention is a dosage form comprising a pharmaceutical composition according to the present invention and optionally one or more pharmaceutical excipients.

In the dosage form of the present invention one or more pharmaceutically acceptable excipient(s), such as fillers, binding agents, lubricants, glidants, anti-sticking agents and disintegrating agents can be employed. Regarding the above-mentioned pharmaceutically acceptable excipients, the application refers to "Lexikon der Hilfsstoffe für Pharmazie, Kosmetik und angrenzende Gebiete", edited by H. P. Fiedler, 4th Edition, Edito Cantor, Aulendorf and earlier editions, and "Hand-book of Pharmaceutical Excipients", Third Edition, edited by Arthur H. Kibbe, American Pharmaceutical Association, Washington, USA, and Pharmaceutical Press, London.

Generally, the comments given above on preferred embodiments of pharmaceutical compositions of the present invention can also apply for the dosage form of the present invention. In particular, the dosage forms of the present invention can be essentially free of crystalline dapagliflozin. The term "essentially" free means that the dosage forms of the present invention do not contain significant amounts of crystalline dapagliflozin. Preferably, the dosage forms of the present invention comprise less than 5 wt.-%, more preferably less than 1 wt.-%, more preferably less than 0.1 wt.-% dapagliflozin in crystalline form, based on the total weight of the dosage form.

In a preferred embodiment of the invention the dose form is adapted to be administered orally or intravenously. The administration of the present dosage form can preferably be applied independently from meals.

A preferred embodiment of the present invention is the use of cyclodextrin, preferably (2-hydroxy)propyl-β-cyclodextrin, for producing a dapagliflozin-containing dosage form.

It was further surprisingly found by the present inventors that, when adding crude dapagliflozin to cyclodextrin, in particular (2-hydroxy)propyl-β-cyclodextrin, under conditions that enable the compounds to form the above described dapagliflozin-cyclodextrin complex, a portion of the drug remained uncomplexed, regardless of any further addition of cyclodextrin, water or ultrasonic treatmemt. Herefrom it may be concluded that only the dapagliflozin can be included in the cavity of the cyclodextrin, but not the impurites of dapagliflozin. The term "crude dapagliflozin" thus can relate to any dapagliflozin, which can contain an impurity, such as any undesired compound. As a consequence, the term "crude" also applies to all kinds of purity grades of dapagliflozin, such as technical grade dapagliflozin.

Thus, the invention can further relate to a method of purification of dapagliflozin, comprising
a) dissolving or dispersing cyclodextrin in a solvent;
b) adding crude dapagliflozin;
c) separating the complexed dapagliflozin from non-complexed residues.

Step c) can preferably be achieved by filtration, or any other method for separation known to the skilled person.

In step (c) the solvent can be removed. It is preferred that the solvent is completely removed. In a preferred embodiment the composition of the present invention comprises an amount of residual solvent of 100 to 1000 ppm, more preferably of 150 to 500 ppm, still more preferably of 200 to 400 ppm.

Within this application the amount of the residual solvent in the composition of the present invention is determined via gas chromatography.

Gas chromatographic conditions for residual solvent analysis:

| | |
|---|---|
| Instrument | Agilent Gas Chromatograph (6890N) equipped with Gerstel Multi Purpose Sampler MPS2 |
| Column | BP-624, 30M x 0.53mm, of 3.0µm |
| Injector | 200°C, Split Ratio 3:1, Total flow: 10.4ml/min |
| Carrier gas | Nitrogen @ 2.0 ml/min [ (constant flow), (Eq. Pressure : 1.45psi) ] |
| Oven | 40°C (hold for 15 min), Ramp 10°C/min upto 160°C (hold for 5 min) |
| Detector | FID @ 250°C |
| Hydrogen | @ 30.0 ml/min |
| Air | @ 300.0 ml/min |
| Make up flow | 28 ml/min |
| Quantity of sample | 100 mg |
| Solvent for dissolving | N,N-Dimethyl formamide |

For the method of purification of dapagliflozin, generally, the steps and the comments given above about preferred embodiments of the method for producing pharmaceutical compositions can be applied.

The subject invention thus further relates to the use of cyclodextrin and, in particular, (2-hydroxy)propyl-β-cyclodextrin in a method of purification of dapagliflozin.

A further subject of the present invention is pharmaceutical composition comprising dapagliflozin, preferably in form of an inclusion complex. In particular, the invention relates to a pharmaceutical composition comprising dapagliflozin characterized in that on introduction to water (at 25 °C) said composition is capable of forming a solution comprised of not less than 5 mg dapagliflozin per ml water. Preferably, the solution comprises 5 mg to 50 mg dapagliflozin, more preferably 6 to 40 mg, still more preferably 8 to 30 mg.

The present invention is illustrated by the following examples.

### EXAMPLES

### Example 1: Preparation of a Dapagliflozin/(2-hydroxy)propyl-β-cyclodextrin Inclusion Complex using Pure Dapagliflozin

9.03 g HPBCD (8.43 g - 6.2 mmol on dry basis, Roquette Frères) were dissolved in 100 ml distilled water. 2.30 g dapagliflozin (5.6 mmol) was added to the solution at 23 °C. The solution was stirred for 15 minutes and additional 25 ml of destilled water were added. After treating the solution in an ultrasonic bath for 7 minutes, the resulting clear solution was frozen and freeze-dried in bulk at 72 m Torr (-52°) for 32 hours.

The resulting pharmaceutical composition can be examined by DSC. The differential scanning calometry (DSC) was performed using the the following parameters:
Start Temperature : 30°C
End Temperature: 300°C
Heating rate : 10°C /min.
Segment gas : Nitrogen
Segment gas flow rate: 50 ml /min.
Sample holder : Aluminum standard 40µl
Sample quantity: 1 - 2 mg

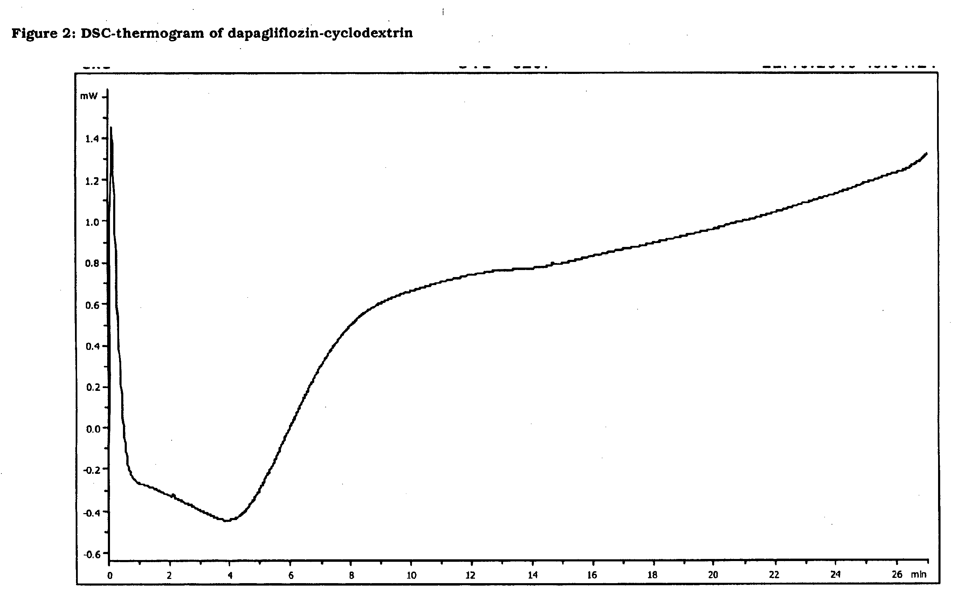

As derivable from figure 2 the DSC-thermogram showed the absence of any crystalline dapagliflozin in the pharmaceutical composition.

### Example 2: Preparation of a Dapagliflozin/(2-hydroxy)propyl-β-cyclodextrin Inclusion Complex using Crude Dapagliflozin

9.03 g HPBCD (8.43 g - 6.2 mmol on dry basis, Roquette Frères) were dissolved in 100 ml distilled water. 2.30 g (5.6 mmol) crude dapagliflozin were added to the solution at 23 °C. The solution was stirred for 15 minutes and additional 25 ml of destilled water were added. After treteating the solution in an ultrasonic bath for 7 minutes, the heterogeneous liquid was filteed through a cellulose nitrate membrane, having a nomimal pore size of 0.22 µm. The resulting clear solution was frozen and freeze-dried in bulk at 72 m Torr (-52°) for 32 hours.

### Example 3: In vitro Solubility Profiles

The effect of cyclodextrins on the aqueous solubility of dapagliflozin was studied in deionised water at 25 ± 2 °C. The solubilizing effect was studied by preparing samples having a cyclodextrin concentration in the range of 1 to 15 wt.%. Due to the relatively low solubility of BCD, the study was performed by adding smaller amounts of cyclodextrin (0.5 - 2 wt.%).

The following cyclodextrins were used:
- β-cyclodextrin (BCD): CYL-2518/2 (Wacker Chemie GmbH)
- (2-hydroxy)propyl-β-cyclodextrin (HPBCD): E0062 (Roquette Frères)
- sulfobutylether-β-cyclodextrin (SBEBCD): 47K040508 (CycloLab)

**Table 1: Solubility of dapagliflozin in deionised water at 25 ± 2 °C in the presence of different cyclodextrins**

| **Applied Cyclodextrin** | **wt.% cyclodextrin** | **Dissolved Dapagliflozin (mg/ml)** |
|---|---|---|
| none | | 1.6 |
| BCD | 0.5 | 3.3 |
| BCD | 1.0 | 4.7 |
| BCD | 1.5 | 6.3 |
| BCD | 2.0 | 7.7 |
| SBEBCD | 1.0 | 3.8 |
| SBEBCD | 5.0 | 10.7 |
| SBEBCD | 10.0 | 13.1 |
| SBEBCD | 15.0 | 12.8 |
| HPGCD | 1.0 | 4.2 |
| HPGCD | 5.0 | 11.3 |
| HPGCD | 10.0 | 14.9 |
| HPGCD | 15.0 | 14.4 |
| HPBCD | 1.0 | 4.4 |
| HPBCD | 2.0 | 7.2 |
| HPBCD | 2.5 | 8.6 |
| HPBCD | 3.0 | 9.7 |
| HPBCD | 4.0 | 12.5 |
| HPBCD | 5.0 | 14.4 |
| HPBCD | 10.0 | 14.1 |
| HPBCD | 15.0 | 14.0 |

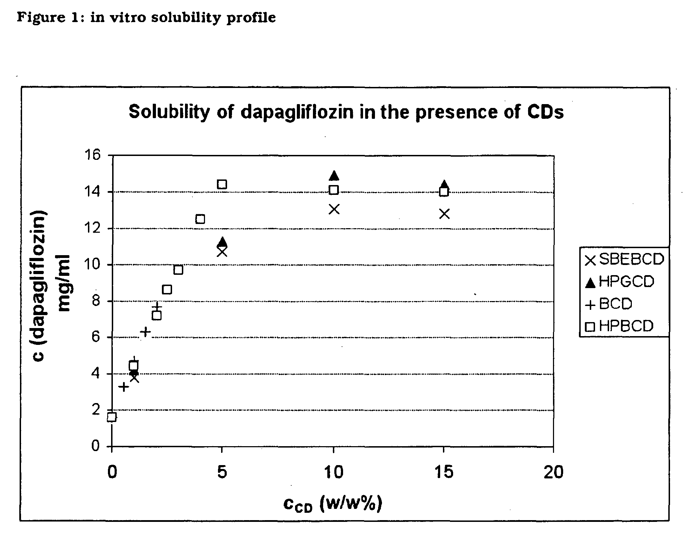

The results are plotted in Figure 1.

The phase solubility studies indicate that the interactions between dapagliflozin and cyclodextrins bring about significant solubility enhancement in deionised water in a cyclodextrin-concentration dependent manner.

### Example 4: Third Component Trial

Literatural examples indicate that the effectiveness of complex formation may be increased upon the addition of hydroxycarboxylic acids, especially citric acid in case the complex forming cyclodextrin or its complex is subject to aggregation. Often this is the case when parent cyclodextrins are applied. Since the selected cyclodextrin (HPBCD) of adequate quality is not likely to aggregate, the addition of a third component is not required. The solubility isotherms of BCD and HPBCD practically coincide within experimental error, indicating that the outer sphere interactions (responsible for aggregation) have no significant effect on the solubility, for example, mainly the cavity size determines the complex stability parameters. As a proof of concept, representative isotherm data were determined in the presence of citric acid (added in equimolar ratio with the pharmacon) and compared to the original dissolved concentrations (Table 2). The data show that the presence of the hydroxycarboxylic acid does not have a significant effect on the solubility of the drug.

**Table 2: Effect of citric acid on the solubility of dapagliflozin in the presence of HPBCD**

| **Dissolving medium** | **Dissolved dapagliflozin (mg/mL)** | **Dissolving medium** | **Dissolved dapagiflozin (mg/mL)** |
|---|---|---|---|
| HPBCD 1.0 % | **4.4** | HPBCD 1.0 % + 28 mg citric acid | **4.7** |
| HPBCD 25 % | **8.6** | HPBCD 25 % + 28 mg citric acid | **8.1** |
| HPBCD 5.0 90 | **14.4** | HPBCD 5.0 % + 28 mg citric acid | **143** |

The phase "solubility isotherms" indicated that HPBCD interacts with dapagliflozin in a 1:1 molar ratio at ambient temperature.

## Claims

1. Pharmaceutical composition comprising cyclodextrin and dapagliflozin.

2. Pharmaceutical composition according to claim 1 in form of an inclusion complex.

3. Pharmaceutical composition according to claim 1 or 2, wherein the molar ratio of cyclodextrin to dapagliflozin is from 0.5 : 1 to 2 : 1, more preferably from 0.8 : 1 to 1.2 : 1, most preferably about 1:1.

4. Pharmaceutical composition according to any of claims 1 to 3, wherein cyclodextrin is (2-hydroxy)propyl-β-cyclodextrin, sulfobutylether-β-cyclodextrin or (2-hydroxy)propyl-γ-cyclodextrin, preferably 2-hydroxy)propyl-β-cyclodextrin.

5. Pharmaceutical composition comprising cyclodextrin and dapagliflozin according to anyone of claims 1 to 4, forming a solid phase being essentially free of crystalline structure.

6. Process for producing a pharmaceutical composition according to claims 1 to 5, comprising the steps of
a) dissolving and/or dispersing cyclodextrin in a solvent;
b) adding dapagliflozin;
c) subjecting the mixture resulting from step b) to a mechanical treatment; and
d) removing the solvent from the reaction mixture, preferably by freeze-drying or spray-drying.

7. Process according to claim 6, wherein the cyclodextrin is (2-hydroxy)propyl-β-cyclodextrin.

8. Process according to claim 6 or 7, wherein pharmaceutical composition is formed in the absence of excipients and/or co-solvents.

9. A pharmaceutical composition obtainable by a process according to any one of claims 6 to 8.

10. Dosage form comprising a pharmaceutical composition according to any one of claims 1 to 5 or 9, and optionally one or more pharmaceutical excipients.

11. Dosage form according to claim 10, being essentially free of crystalline dapagliflozin.

12. Dosage form according to claim 10 or 11, wherein the dosage form is adapted to be administered orally or intravenously.

13. Use of cyclodextrin, preferably (2-hydroxy)propyl-β-cyclodextrin, for producing a dapagliflozin-containing dosage form.

14. Method of purification of dapagliflozin, comprising the steps of
a) dissolving or dispersing cyclodextrin in a solvent;
b) adding crude dapagliflozin;
c) separating the complexed dapagliflozin from non-complexed residues. preferably by filtration.

15. A pharmaceutical composition or dosage form comprising dapagliflozin **characterized in that** on introduction to water said composition is capable of forming a solution comprised of not less than 5 mg dapagliflozin per ml water.

## Patentansprüche

1. Pharmazeutische Zusammensetzung umfassend Cyclodextrin und Dapagliflozin.

2. Pharmazeutische Zusammensetzung nach Anspruch 1 in Form eines Einschlusskomplexes.

3. Pharmazeutische Zusammensetzung nach Anspruch 1 oder 2, wobei das molare Verhältnis von Cyclodextrin zu Dapagliflozin 0,5 : 1 bis 2 : 1, mehr bevorzugt von 0,8 : 1 bis 1,2 : 1, am meisten bevorzugt etwa 1 : 1 beträgt.

4. Pharmazeutische Zusammensetzung nach einem der Ansprüche 1 bis 3, wobei Cyclodextrin (2-Hydroxy)propyl-β-cyclodextrin, Sulfobutylether-β-cyclodextrin oder (2-Hydroxy)propyl-β-cyclodextrin ist, bevorzugt (2-Hydroxy)propyl-β-cyclodextrin.

5. Pharmazeutische Zusammensetzung umfassend Cyclodextrin und Dapagliflozin nach einem der Ansprüche 1 bis 4, welche eine feste Phase bildet, welche im Wesentlichen frei von kristalliner Struktur ist.

6. Verfahren zur Herstellung einer pharmazeutischen Zusammensetzung nach den Ansprüchen 1 bis 5, umfassend die Schritte
a) Auflösen und/oder Dispergieren von Cyclodextrin in einem Lösungsmittel;
b) Zugeben von Dapagliflozin;
c) Unterziehen der aus Schritt b) resultierenden Mischung einer mechanischen Behandlung;
und
d) Entfernen des Lösungsmittels von der Reaktionsmischung, bevorzugt durch Gefriertrocknung oder Sprühtrocknung.

7. Verfahren nach Anspruch 6, wobei das Cyclodextrin (2-Hydroxy)propyl-β-cyclodextrin ist.

8. Verfahren nach Anspruch 6 oder 7, wobei die pharmazeutische Zusammensetzung unter Abwesenheit von Hilfsstoffen und/oder zusätzlichem Lösungsmittel geformt wird.

9. Pharmazeutische Zusammensetzung, erhältlich durch ein Verfahren nach einem der Ansprüche 6 bis 8.

10. Darreichungsform umfassend eine pharmazeutische Zusammensetzung nach einem der Ansprüche 1 bis 5 oder 9 und optional einen oder mehrere pharmazeutische Hilfsstoffe.

11. Darreichungsform nach Anspruch 10, welche im Wesentlichen frei von kristallinem Dapagliflozin ist.

12. Darreichungsform nach Anspruch 10 oder 11, wobei die Darreichungsform geeignet für eine orale oder intravenöse Verabreichung ist.

13. Verwendung von Cyclodextrin, bevorzugt (2-Hydroxy)propyl-β-cyclodextrin, für die Herstellung einer Dapagliflozin enthaltenden Darreichungsform.

14. Verfahren zur Reinigung von Dapagliflozin, umfassend die Schritte
a) Auflösen oder Dispergieren von Cyclodextrin in einem Lösungsmittel,
b) Zugabe von rohem Dapagliflozin;
c) Trennung von komplexiertem Dapagliflozin und nicht-komplexierten Rückständen, bevorzugt durch Filtration.

15. Eine pharmazeutische Zusammensetzung oder Darreichungsform umfassend Dapagliflozin, **dadurch gekennzeichnet, dass** bei der Zugabe von Wasser die besagte Zusammensetzung eine Lösung bilden kann, welche aus nicht weniger als 5 mg Dapagliflozin pro ml Wasser besteht.

## Revendications

1. Composition pharmaceutique comprenant de la cyclodextrine et de la dapagliflozine.

2. Composition pharmaceutique selon la revendication 1, sous la forme d'un complexe d'inclusion.

3. Composition pharmaceutique selon la revendication 1 ou 2, dans laquelle le rapport molaire de la cyclodextrine sur la dapagliflozine est de 0,5/1 à 2/1, de manière davantage préférée de 0,8/1 à 1,2/1, idéalement d'environ 1/1.

4. Composition pharmaceutique selon l'une quelconque des revendications 1 à 3, dans laquelle la cyclodextrine est la (2-hydroxy)propyl-β-cyclodextrine, la sulfobutyléther-β-cyclodextrine ou la (2-hydroxy)propyl-γ-cyclodextrine, de préférence la (2-hydroxy)propyl-β-cyclodextrine.

5. Composition pharmaceutique comprenant de la cyclodextrine et de la dapagliflozine selon l'une quelconque des revendications 1 à 4, formant une phase solide sensiblement dépourvue de structure cristalline.

6. Procédé de production d'une composition pharmaceutique selon l'une quelconque des revendications 1 à 5, comprenant les étapes de :
a) dissolution et/ou dispersion de la cyclodextrine dans un solvant ;
b) ajout de dapagliflozine ;
c) soumission du mélange résultant de l'étape b) à un traitement mécanique ; et
d) élimination du solvant du mélange réactionnel, de préférence par lyophilisation ou par séchage par pulvérisation.

7. Procédé selon la revendication 6, dans lequel la cyclodextrine est la (2-hydroxy)propyl-β-cyclodextrine.

8. Procédé selon la revendication 6 ou 7, dans lequel la composition pharmaceutique est formée en l'absence d'excipient et/ou de cosolvant.

9. Composition pharmaceutique pouvant être obtenue par un procédé selon l'une quelconque des revendications 6 à 8.

10. Forme galénique comprenant une composition pharmaceutique selon l'une quelconque des revendications 1 à 5 ou 9, et facultativement un ou plusieurs excipients pharmaceutiques.

11. Forme galénique selon la revendication 10, étant sensiblement dépourvue de dapagliflozine cristalline.

12. Forme galénique selon la revendication 10 ou 11, dans laquelle la forme galénique est conçue pour être administrée par voie orale ou intraveineuse.

13. Utilisation de cyclodextrine, de préférence de (2-hydroxy)propyl-β-cyclodextrine, pour la production d'une forme galénique contenant de la dapagliflozine.

14. Procédé de purification de dapagliflozine, comprenant les étapes de :
a) dissolution et/ou dispersion de la cyclodextrine dans un solvant ;
b) ajout de dapagliflozine brute ;
c) séparation de la dapagliflozine complexée des résidus non complexés, de préférence par filtration.

15. Composition pharmaceutique ou forme galénique comprenant de la dapagliflozine **caractérisée en ce que** lors de l'introduction dans l'eau, ladite composition est capable de former une solution ne comprenant pas moins de 5 mg de dapagliflozine par mL d'eau.
